# EUROPÄISCHE PATENTSCHRIFT

(11) **EP 1 585 712 B1**
(45) Veröffentlichungstag und Bekanntmachung des Hinweises auf die Patenterteilung: **17.09.2014**
(21) Anmeldenummer: 03799530.5
(22) Anmeldetag: 03.12.2003
(51) Int. Cl.: C07C 2/28, C07C 7/148, C07C 41/06, C07C 41/42

(54) **VERFAHREN ZUR HERSTELLUNG VON BUTENOLIGOMEREN UND TERT.-BUTYLETHERN AUS ISOBUTENHALTIGEN C4-STRÖMEN**
METHOD FOR PRODUCING BUTENE OLIGOMERS AND TERT-BUTYL ETHERS FROM C4 FLOWS CONTAINING ISOBUTENE
PROCEDE DE PREPARATION D'OLIGOMERES BUTENE ET DE TERT-BUTYLETHERS A PARTIR DE FLUX C4 CONTENANT DE L'ISOBUTENE

(30) Priorität: 23.01.2003 DE 10302457
(43) Veröffentlichungstag der Anmeldung: 19.10.2005
(73) Patentinhaber: Evonik Degussa GmbH, 45128 Essen (DE)
(72) Erfinder: PETERS, Udo, 45770 Marl (DE); REUSCH, Dieter, 45772 Marl (DE); BECKMANN, Andreas, 45657 Recklinghausen (DE); RÖTTGER, Dirk, 45657 Recklinghausen (DE); PRAEFKE, Jochen, 45770 Marl (DE)
(74) Vertreter: Hirsch, Hans-Ludwig
(86) Internationale Anmeldenummer: PCT/EP2003/050930
(87) Internationale Veröffentlichungsnummer: WO 2004/065338

(56) Entgegenhaltungen:
- EP-A- 0 081 041
- EP-A- 1 074 534
- EP-A- 1 199 296
- WO-A-02/064531
- DE-A- 2 944 457
- DATABASE WPI Section Ch, Week 198521 Derwent Publications Ltd., London, GB; Class E17, AN 1985-124697 XP002278112 & JP 60 058932 A (MITSUBISHI GAS CHEM CO INC), 5. April 1985 (1985-04-05)

## Beschreibung

Die Erfindung betrifft ein Verfahren zur Herstellung von Butenoligomeren und tert.-Butylethern aus C₄-Kohlenwasserstoffströmen, durch Umsetzen des Isobutens zu Butenoligomeren, insbesondere Isobutenoligomeren und tert.-Butylethern und deren Entfernung aus den C₄-Strömen.

Isobuten, lineare Butene und deren Folgeprodukte werden in großen Mengen aus technischen C₄-Schnitten, beispielsweise dem C₄-Schnitt aus Steamcrackern oder FCC-Einheiten, gewonnen. Diese Gemische bestehen im Wesentlichen aus Butadien, den Monoolefinen Isobuten, 1-Buten und den beiden 2-Butenen sowie den gesättigten Kohlenwasserstoffen Isobutan und n-Butan. Wegen der geringen Siedepunktsunterschiede der Inhaltsstoffe und deren geringen Trennfaktoren ist eine destillative Aufarbeitung schwierig und nicht wirtschaftlich. Die Gewinnung von linearen Butenen und von anderen Produkten erfolgt daher meistens durch eine Kombination von chemischen Umsetzungen und physikalischen Trennoperationen.

Abhängig davon, ob die Butene einen hohen oder geringen Anteil an 1-Buten enthalten, werden die C₄-Schnitte unterschiedlich aufgearbeitet. Der erste Schritt, den dabei alle Aufarbeitungsvarianten gemeinsam haben, ist die Entfernung des größten Teils des Butadiens. Kann Butadien gut vermarktet werden oder besteht ein Eigenverbrauch, wird es durch Extraktion oder Extraktivdestillation abgetrennt. Im anderen Fall wird es bis zu einer Restkonzentration von circa 2000 Massen-ppm selektiv zu linearen Butenen hydriert. Zurück bleibt in beiden Fällen ein Kohlenwasserstoffgemisch (sogenanntes Raffinat I oder hydriertes Crack-C₄), das neben den gesättigten Kohlenwasserstoffen n-Butan und Isobutan die Olefine Isobuten, 1-Buten und 2-Butene (cis und trans) enthält.

Ist die Herstellung von Isobuten und 2-Buten beziehungsweise ein Gemisch linearer Butene mit hohem 2-Butengehalt das Ziel, wird folgender Aufarbeitungsgang bevorzugt: C₄-Ströme, die typischerweise nicht mehr als 1 % Butadien enthalten (C₄-Strom aus FCC (Fluid catalytic cracker), Raffinat I oder hydriertes Crack-C₄), werden hydriert und hydroisomerisiert, das heißt, es wird vorhandenes Butadien bis zu einem Restgehalt von unter 5 ppm selektiv hydriert und gleichzeitig 1-Buten zu 2-Butenen isomerisiert. Die Gleichgewichtslage zwischen 1-Buten und den beiden 2-Butenen liegt bei 80 °C bei circa 1 : 17, also weit auf der Seite der 2-Butene. Aus dem Hydroisomerisierungsgemisch kann wegen der geringen Siedepunktsdifferenzen nur ein Gemisch aus Isobuten, 1-Buten und Isobutan als Kopfprodukt gewonnen werden, das in üblicher Weise aufgearbeitet werden kann. Als Sumpfprodukt wird ein Isobuten-freies Gemisch erhalten, das aus 2-Butenen und n-Butan besteht. Wird die Hydroisomerisierung in einer Reaktivdestillationskolonne durchgeführt, kann ein nahezu 1-Buten-freies Isobuten erhalten werden.

Wenn 1-Buten eines der Zielprodukte ist, kann wie folgt weiter vorgegangen werden: aus Raffinat I oder hydriertem Crack-C₄ wird durch chemische Umsetzung Isobuten entfernt. Nach der Entfernung des Isobutens bleibt ein Kohlenwasserstoffgemisch (Raffinat II), das die linearen Butene und die gesättigten Kohlenwasserstoffe Isobutan und n-Butan enthält. Dieses Gemisch kann destillativ weiter aufgetrennt werden, beispielsweise in Isobutan und 1-Buten und ein Gemisch aus den beiden 2-Butenen und n-Butan. Aus der 1-Buten-haltigen Fraktion kann in weiteren Destillationsschritten 1-Buten in hoher Reinheit erhalten werden, welches nur noch geringe Mengen an Isobutan enthält. Dies ist notwendig, da 1-Buten im großen Maße als Comonomer in der Ethylenpolymerisation eingesetzt wird, wo Isobuten-Verunreinigungen unerwünscht sind. Typische Spezifikationen von 1-Buten beschränken daher den Gehalt an Isobuten im 1-Buten auf unter 1000 ppm.

Die chemische Umsetzung des Isobutens kann mit Wasser zu tert.-Butylalkohol (TBA) durchgeführt werden. Dieser Weg ist wegen der geringen Löslichkeit von Wasser in C₄-Kohlenwasserstoffe aufwändig und somit teuer. Zudem lässt sich eine vollständige Isobutenabtrennung kaum realisieren.

Eine andere Möglichkeit besteht darin, das Isobuten zu oligomerisieren und das Oligomerisat abzutrennen. Nachteilig dabei ist, dass bei der vollständigen Isobuten-Entfernung durch Oligomerisation auch ein großer Teil der vorliegenden linearen Butene zu Co- oder Homooligomeren umgesetzt wird. Ein weiterer Nachteil ist die teilweise Isomerisierung von 1-Buten zu den 2-Butenen.

Eine weitere Möglichkeit zur Abtrennung von Isobuten ist dessen Umsetzung mit Alkoholen, beispielsweise Methanol oder Ethanol, zu den entsprechenden tertiären Butylethern.

Das technisch bedeutendste Verfahren ist die Umsetzung von Isobuten mit Methanol zu Methyl-tert.-butylether (MTBE), das hauptsächlich als Kraftstoffzusatz große Verwendung findet.

Die Verwendung von tertiären Butylethern, insbesondere MTBE, als Octanzahlverbesserer in Ottokraftstoffen wird wegen in den USA angetretenen Grundwasserkontaminationen zunehmend kritisch gesehen. Es ist nicht auszuschließen, dass die derzeitigen und künftigen Diskussionen zur Einschränkung der Verwendung von tertiären Butylethern in Kraftstoffen führen.

Da die Verwendung von Butylethern als Kraftstoffzusatz in Zukunft nicht mehr die Gesamtisobutenmenge aufnehmen wird, besteht Interesse an anderen Methoden, Isobuten aus C₄-Gemischen unter chemischer Umwandlung zu entfernen. Die Herstellung von Oligomeren des Isobutens und deren Abtrennung aus dem Rest.-C₄-Gemisch ist hierzu eine vielversprechende Möglichkeit.

In EP 0 048 893 wird ein Verfahren zur gleichzeitigen Herstellung von Isobutenoligomeren und Alkyl-tert.-butylether aus C₄-Schnitten in einem Reaktor dargelegt. Als Katalysator wird ein saures Ionenaustauscherharz, das teilweise mit Metallen der siebten und achten Nebengruppe des Periodensystems der Elemente in elementarer Form (Oxidationsstufe 0) belegt ist, verwendet. Die Produkte und die nicht umgesetzten C₄-Kohlenwasserstoffe werden destillativ getrennt. Bei diesem Verfahren gehen circa 8 % der linearen Butene durch Oligomerisierung verloren. Der Verlust an 1-Buten liegt bei 7 %. Der Hauptnachteil dieses Verfahren ist jedoch, dass kein vollständiger Isobutenumsatz erreicht wird, sodass der Isobutengehalt in der abgetrennten C₄-Kohlenwasserstoff-Fraktion zu hoch ist, um daraus ein spezifikationsgerechtes 1-Buten zu gewinnen.

US 5,723,687 beschreibt ebenfalls ein Verfahren zur gleichzeitigen Herstellung von Isobutenoligomeren und MTBE oder ETBE durch Umsetzung eines C₄-Schnitts, beispielsweise Raffinat I, in Anwesenheit von Methanol oder Ethanol an einem sauren Ionenaustauscherharz in einem Reaktor. Die Verluste an 1-Buten dabei sind nicht offengelegt. Nachteilig bei diesem Verfahren ist der geringe Isobuten-Umsatz, der gemäß den Beispielen nur zwischen 78 und 94 % liegt

Der Einsatz von modifizierten Ionenaustauschern zur Oligomerisierung von Isobuten ist in DE 101 13 381 offenbart. Hier werden allerdings Isobuten-haltige Ströme eingesetzt, die praktisch keine weiteren ungesättigten Verbindungen wie n-Butene enthalten.

In DB 29 44 457 wird ein Verfahren zur gleichzeitigen Herstellung von Isobutenoligomeren und MTBE aus C₄-Koblenwasserstoffselmiften, beispielsweise Raffinat I, beschrieben. Dabei wird in einem ersten Reaktionsschritt 50 bis 90 % des Isobutens an einem stark sauren Katalysator oligomerisiert. Das restliche Isobuten wird in einem zweiten Reaktionsschritt mit Methanol zu MTBE umgesetzt. Die Reaktionsprodukte und die nicht umgesetzten C₄ Kohlenwasserstoffe werden destillativ getrennt. Dieses Verfahren hat folgende Nachteile:
a) Über 30 % der linearen Butene gehen durch Oligomerisierung verloren
b) Die Verluste an 1-Buten durch Oligomerisierung und Isomerisierung betragen mehr als 33 %.
c) Im verbleibenden C₄-Kohlenwasserstoffgemisch liegt der Isobutengehalt bei über 0,7 %, d. h., aus diesem Gemisch kann kein hochreines 1-Buten mit einem Isobutengehalt kleiner 1000 ppm hergestellt werden.

Ein einstufiges Verfahren zur gekoppelten Herstellung von Butenoligomeren und MTBE aus Isobuten-haltigen C₄-Strömen durch teilweise Oligomerisierung der Isobuten-haltigen Ströme und anschließende sauerkatalytische Veretherung des restlichen Isobutens zu MTBE ist aus DE-A-2944457 bekannt.

EP-A-1199296 lehrt die Entfernung von Isobuten aus C₄-Strömen durch sauerkatalytische Veretherung des Isobutens mit einem Alkohl zu tert.-Butylethern in mindestens zwei Reaktionsstufen durchzuführen, wobei die letzte Reaktionsstufe als eine Reaktivdestillation ausgeführt wird. Es lässt sich dadurch ein hochreines 1-Buten aus dem von Isobuten befreiten Gemisch erhalten.

Aus der WO-A-02064531 ist ein Verfahren zur Herstellung von hochreinem Diisobuten durch Oligomerisierung von Isobuten-haltigen Kohlenwasserstoffgemischen an einem festen sauren Ionenaustauscherharz bekannt, bei dem das saure Ionenaustauscherharz Sulfonsäuregruppen umfasst, deren Protonen zu 50 bis 80 % gegen Metallionen ausgetauscht sind.

EP-A-1074534 beschreibt die Durchführung einer Oligomerisierung von Isobuten in Anwesenheit von MTBE oder Methanol, wobei das Molverhältnis des MTBE bzw. des Methanols zu Isobuten mehr als 0.1 beträgt.

EP-A-0081041 offenbart die katalytische Hydrierung von mehrfach ungesättigten Kohlenwasserstoffen in einem C₄-Strom in Gegenwart von Kohlenmonoxid in einer Konzentration von 0.05 bis 20 wppm.

Da die oben genannten Verfahren hinsichtlich der 1-Buten-Ausbeute und/oder des Isobutengehaltes im abgetrennten C₄-Kohlenwasserstoffgemisches (Raffinat II) nicht zufriedenstellend sind, bestand die Aufgabe, ein verbessertes Verfahren zu entwickeln.

Es wurde nun gefunden, dass das Isobuten aus einem weitgehend Butadien-freien C₄-Kohlenwasserstoffstrom mit nur geringen Verlusten an linearen Butenen vollständig entfernt werden kann, indem ein Teil des Isobutens in einem ersten Reaktionsschritt an sauren Katalysatoren, deren Aktivität durch Zugabe von Moderatoren oder Ionenaustausch modifiziert wurde, oligomerisiert wird und dass in einem zweiten Reaktionsschritt das restliche Isobuten durch Umsetzung mit Alkohol zu einem tert.-Butylether in einer Reaktivdestillationskolonne entfernt wird.

Im Gegensatz zu den bekannten Verfahren geht nur ein geringer Teil des im Gemisch vorliegenden 1-Butens durch Isomerisierung zu den 2-Butenen verloren.

Gegenstand der Erfindung ist demnach ein Verfahren nach Anspruch 1.

Butenoligomere im Sinne der vorliegenden Erfindung sind insbesondere Isobutenoligomere wie Di-, Tri- oder Tetramere des Isobutens. In geringem Maße können diese auch Cooligomeren mit oder von 1- oder 2-Butenen enthalten.

Bevorzugt enthalten die erfindungsgemäß hergestellten Butenoligomere mehr als 90 % Isobutenoligomere, besonders bevorzugt weist die Oligomermischung
90 - 97 % Isobutenoligomeren
1 - 8 % Iso- und n-Buten Cooligomeren und
0,1 - 2 % n-Butenoligomeren, auf.

Mit Hilfe des erfindungsgemäßen Verfahrens können aus C₄-Strömen nicht nur die Wertprodukte tert.-Butylether und Isobuten-Oligomere, sondern auch Isobuten-freie Restströme, die sich zur Herstellung von 1-Buten eignen, produziert werden. Die Isobutenfreien Restströme enthalten neben den nicht umgesetzten Bestandteilen des C₄-Feed (in der Regel C₄-Aliphate) nur noch n-Butene, d. h. 1-Buten, cis-2-Buten und trans-2-Buten. Aus dieser Mischung kann 1-Buten destillativ abgetrennt werden (Das dabei ggf. zusammen mit dem 1-Buten abgetrennte i-Butan kann in einem zweiten Destillationsschritt von 1-Buten getrennt werden). Die zurückbleibenden 2-Butene, die ggf. noch 1-Buten enthalten können, sind wiederum begehrte Einsatzstoffe zur Herstellung von linearen Butenoligomeren wo die Anwesenheit von Isobuten zu unerwünscht hohen Verzweigungsgraden führen würde.

Geeignete Isobuten-haltige C₄-Ströme sind beispielsweise Leichtbenzinfraktionen aus Raffinerien, C₄-Fraktionen aus Crackern (beispielsweise Steamcracker, Hydrocracker, Katcracker), Gemische aus Fischer-Tropsch-Synthesen, Gemische aus der Dehydrierung von Butanen, Gemische aus Skelettisomerisierung linearer Butene und Gemische, entstanden durch Metathese von Olefinen. Diese Techniken sind in der Fachliteratur beschrieben, K.Weissermel, H.J. Arpe, Industrielle Organische Chemie, Wiley-VCH, 5. Auflage, 1998, Seite 23-24; 65-99; 122-124.

Bevorzugt eingesetzt werden C₄-Fraktionen aus Steamcrackern, die primär zur Produktion von Ethen und Propen betrieben werden und in denen als Rohstoffe beispielsweise Raffineriegase, Naphtha, Gasöl, LPG (liquified petroleum gas) und NGL (natural gas liquid) eingesetzt werden oder Katcrackern. Die als Nebenprodukt anfallenden C₄-Schnitte enthalten je nach Crack-Verfahren unterschiedliche Mengen an Isobuten. Weitere Hauptbestandteile sind 1,3-Butadien, 1-Buten, c-2-Buten, t-2-Buten, n-Butan und i-Butan. Typische Isobuten-Gehalte in der C₄-Fraktion liegen bei C₄-Fraktionen aus Steam-Crackern bei 18 bis 35%, bei FCC-Katcrackern bei 10 bis 20 %.

Für das erfindungsgemäße Verfahren ist es vorteilhaft, mehrfach ungesättigte Kohlenwasserstoffe wie 1,3-Butadien aus dem Einsatzgemisch zu entfernen. Dies kann nach bekannten Verfahren, beispielsweise durch Extraktion, Extraktivdestillation oder Komplexbildung erfolgen (vgl. K.Weissermel, H.J. Arpe, Industrielle Organische Chemie, Wiley-VCH, 5. Auflage, 1998, Seite 119-121).

Eine Alternative zur Abtrennung der mehrfach ungesättigten Kohlenwasserstoffe ist eine selektive chemische Umsetzung. So kann beispielsweise 1,3-Butadien selektiv zu linearen Butenen hydriert werden, wie z. B. beschrieben in EP 0 523 482. Auch durch selektive Umsetzungen des 1,3-Butadiens, zum Beispiel Dimerisierung zum Cyclooctadien, Trimerisierung zum Cyclododecadien, Polymerisations- oder Telomerisationsreaktionen, kann das 1,3-Butadien zumindest teilweise entfernt werden. Wurde ein Crack-C₄-Schnitt als Rohstoff eingesetzt, bleibt in allen Fällen ein Kohlenwasserstoffgemisch (Raffinat I oder hydriertes Crack-C₄ (HCC₄)) zurück, das hauptsächlich die gesättigten Kohlenwasserstoffe, n-Butan und Isobutan und die Olefine Isobuten, 1-Buten und 2-Butene enthält.

Bevorzugt werden im erfindungsgemäßen Verfahren die in den Isobuten-haltigen C₄-Strömen enthaltenen mehrfach ungesättigten Kohlenwasserstoffe vor der Oligomerisierung in Stufe a) katalytisch hydriert. Bei den mehrfach ungesättigten Kohlenwasserstoffen handelt es sich hauptsächlich um 1,3-Butadien; 1,2-Butadien, Butenin und 1-Butin sind wenn in deutlich geringerer Menge enthalten. Die Hydrierung kann in einem ein- oder mehrstufigen Hydrierprozess in der Flüssigphase an einem Palladiumkontakt erfolgen. Zu Absenkung des Gehalts an 1,3-Butadien unter 1000 ppm wird dabei in der letzten Stufe der Hydrierung mit Zusatz eines Moderators gearbeitet, der die Selektivität des Palladiumkontakts erhöht. Bevorzugt wird als Moderator Kohlenmonoxid eingesetzt, das in einem Anteil von 0.05 bis 100 Gew. ppm zugesetzt wird. Der Gehalt an mehrfach ungesättigten Kohlenwasserstoffen sollte im Zulauf zu dieser Stufe unter 1 %, bevorzugt unter 0.5 %, betragen. In der Literatur ist diese Art der Selektivhydrierung von Restgehalten an 1,3-Butadien unter der Bezeichnung SHP (selective hydrogenation process) bekannt (vergleiche EP 0 081 041; Erdöl, Kohle, Erdgas, Petrochem. 1986, 39, 73).

Sind in den Isobuten-haltigen C₄-Strömen Mengen größer 1 % an mehrfach ungesättigten Kohlenwasserstoffe wie 1,3-Butadien enthalten, werden sie in vorgeschalteten Hydrierungen umgesetzt. Diese Hydrierungen werden bevorzugt in der Flüssigphase an einem Palladiumkontakt durchgeführt. Je nach Gehalt an ungesättigten Kohlenwasserstoffen kann die Hydrierung in mehreren Stufen durchgeführt werden. Zur Umsetzung von Crack-C₄ aus einem Steam-Cracker mit einem Gehalt an 1,3-Butadien von typischerweise 38 - 45 % hat sich eine zweistufige Ausführung der Hydrierung bewährt. Dabei können einzelne oder alle Stufen mit einer teilweisen Produktrückführung ausgestattet sein. Im Austrag sind so Konzentrationen an 1,3-Butadien kleiner 1 % erhältlich, so dass eine weitere Umsetzung in einer SHP Stufe erfolgen kann.

Die in dem erfindungsgemäßen Verfahren eingesetzten Kohlenwasserstoffmischungen mit Isobuten und linearen Butenen weisen bevorzugt die folgenden Zusammensetzungen auf:

| **Tabelle 1** | | | | | | |
|---|---|---|---|---|---|---|
| **Typische Zusammensetzungen von Kohlenwasserstoffmischungen, die im erfindungsgemäßen Verfahren eingesetzt werden.** | | | | | | |
| | Steamcracker | | Steamcracker | | Katcracker | |
| Komponente | HCC₄ | HCC₄ / SHP | Raff. I | Raff. I / SHP | CC₄ | CC₄ / SHP |
| Isobutan [Gew.%] | 1-4.5 | 1-4.5 | 1.5-8 | 1.5-8 | 37 | 37 |
| n-Butan [Gew.%] | 5-8 | 5-8 | 6-15 | 6-15 | 13 | 13 |
| t-Buten [Gew.%] | 18-21 | 18-21 | 7-10 | 7-10 | 12 | 12 |
| 1-Buten [Gew.%] | 35-45 | 35-45 | 15-35 | 15-35 | 12 | 12 |
| Isobuten [Gew.%] | 22-28 | 22-28 | 33-50 | 33-50 | 15 | 15 |
| c-Buten [Gew.%] | 5-9 | 5-9 | 4-8 | 4-8 | 11 | 11 |
| 1,3-Butadien [ppm] | 500-5000 | 0-50 | 50-8000 | 0-50 | <10000 | 0-50 |
| Erläuterung | | | | | | |
| - HCC₄: typisch für eine C₄ Mischung, die aus dem Crack-C₄ eines Steamcrackers (High Severity) nach der Hydrierung des 1,3-Butadiens ohne zusätzliche Moderation des Katalysators erhalten wird. | | | | | | |
| - HCC₄ / SHP: Zusammensetzung HCC₄, bei dem Reste an 1,3-Butadien in einer SHP weiter reduziert wurden. | | | | | | |
| - Raff. I (Raffinat I): typisch für eine C₄ Mischung, die aus dem Crack-C₄ eines Steamcrackers (High Severity) nach der Abtrennung des 1,3-Butadiens, beispielsweise durch eine NMP-Extraktivrektifikation, erhalten wird. | | | | | | |
| - Raff. I / SHP: Zusammensetzung Raff. I, bei dem Reste an 1,3-Butadien in einer SHP weiter reduziert wurden. | | | | | | |
| - CC₄: typische Zusammensetzung eines Crack-C₄, das aus einem Katcracker erhalten wird. | | | | | | |
| - CC₄ / SHP: Zusammensetzung CC₄, bei dem Reste an 1,3-Butadien in einer SHP weiter reduziert wurden. | | | | | | |

Das Raffinat I bzw. HCC₄ ist, neben anderen, ein bevorzugte eingesetztes Isobuten-haltiges Kohlenwasserstoffgemisch im Rahmen dieser Erfindung. Da Anlagen zur Aufarbeitung von C₄-Kohlenwasserstoffen in der Regel als Strang (Verbund mehrerer Anlagen) aufgebaut sind, ist es jedoch möglich, dass das Raffinat I bzw. HCC₄ vor dem Eintritt in das erfindungsgemäße Verfahren eine oder mehrere andere Prozessstufen durchläuft. Auf diese Weise kann jeweils ein individuell angepasstes Gesamtkonzept zur Aufarbeitung mit dem entsprechenden Produktportfolio realisiert werden.

Typische Prozessstufen, die den erfindungsgemäßen Verfahren vorgelagert sein können, sind Wasserwäsche, Reinigung an Adsorbern, Selektivhydrierung, TBA-Synthese, Trocknung und Destillation.

### Wasserwäsche

Durch eine Wasserwäsche können hydrophile Komponenten aus dem Kohlenwasserstoffgemisch, enthaltend Isobuten und lineare Butene, ganz oder teilweise entfernt werden, beispielsweise Stickstoffkomponenten. Beispiele für Stickstoffkomponenten sind Acetonitil oder N-Methylpyrrolidon (die z. B. aus einer 1,3-Butadien-Extraktivdestilltion stammen können). Auch Sauerstoffverbindungen (z. B. Aceton aus FCC-Cracker) können zum Teil über eine Wasserwäsche entfernt werden. Der Isobuten-haltige Kohlenwasserstoffstrom ist nach einer Wasserwäsche mit Wasser gesättigt. Um eine Zweiphasigkeit in den nachfolgenden Prozessschritten im Reaktor zu vermeiden, sollte dort die Reaktionstemperatur um ca. 10 °C über der Temperatur der Wasserwäsche liegen.

### Adsorber

Adsorber werden eingesetzt, um Verunreinigungen zu entfernen. Dies kann beispielsweise vorteilhaft sein, wenn in einem der Prozessschritte Edelmetallkatalysatoren zum Einsatz kommen. Oftmals werden Stickstoff oder Schwefelverbindungen über vorgeschaltete Adsorber entfernt. Beispiele für Adsorber sind Aluminiumoxide, Molekularsiebe, Zeolithe, Aktivkohle, mit Metallen imprägnierte Tonerden. Adsorber werden von diversen Firmen vertrieben, beispielsweise der Firma Alcoa (Selexsorb^{®}).

### Selektivhydrierung (SHP)

Noch in geringen Mengen enthaltene mehrfach ungesättigte Verbindungen, insbesondere 1,3-Butadien, werden durch weitere selektive Hydrierung (vergleiche EP 0 081 041; Erdöl, Kohle, Erdgas, Petrochem. 1986, 39, 73) weiter verringert. Der SHP kann sowohl als Teil einer Butadienhydrierung (siehe oben), oder als eigenständiger Prozessschritt betrieben werden.

### TBA-Synthese

Teile des Isobutens können mit Wasser zu tert.-Butanol (TBA) umgesetzt werden. Verfahren zur Herstellung von TBA aus Isobuten-haltigen Kohlenwasserstoffmischungen sind Stand der Technik (vergleiche zum Beispiel Erdöl, Erdgas, Kohle, 1987, 103, 486). TBA wird beispielsweise als Lösungsmittel eingesetzt, wird aber auch durch Rückspaltung zu Isobuten und Wasser zur Darstellung von hochreinem Isobuten verwendet.

### Trocknung

Im Isobuten-haltigen Kohlenwasserstoffgemisch gegebenenfalls enthaltenes Wasser, das beispielsweise aus der Wasserwäsche oder der TBA-Synthese stammen kann, kann durch bekannte Verfahren zur Trocknung entfernt werden. Geeignete Verfahren sind beispielsweise die destillative Abtrennung des Wassers als Azeotrop. Dabei kann oftmals ein Azeotrop mit enthaltenen C₄-Kohlenwasserstoffen ausgenutzt werden oder es werden Schleppmittel zugesetzt.

Die Trocknung des Kohlenwasserstoffgemisches kann aus diversen Gründen vorteilhaft sein, beispielsweise Verringerung der Bildung von Alkoholen (hauptsächlich tert-Butylalkohol) in der Oligomerisierung/Verätherung, Verhinderung einer (ungesteuerten) Wassermoderation in der Butenoligomerisierung, Vermeidung von technischen Problemen durch Abscheidung von Wasser oder Eis bei niedrigen Temperaturen (z. B. Zwischenlagerung).

### Destillation

Destillationsschritte können beispielsweise genutzt werden, um Verunreinigungen abzutrennen (beispielsweise Leichtsieder wie C3-Kohlenwasserstoffe, Schwersieder wie C₅-Kohlenwasserstoffe) oder um Fraktionen mit unterschiedlichen Isobutenkonzentrationen zu erhalten. Dies kann sowohl direkt mit dem Raffinat I bzw. dem HCC₄ erfolgen oder nachdem eine oder mehrere andere Prozessstufen durchlaufen wurden. Durch direkte Destillation des Raffinats I bzw. des HCC₄s ist beispielsweise eine Trennung in eine an 2-Butenen und n-Butan verarmte, Isobuten reichere Fraktion möglich.

### Erfindungsgemäße Oligomerisierung gemäß Stufe a

Die partielle Oligomerisierung in Stufe a) des Isobutens kann prinzipell homogen, d. h. unter Verwendung von im Reaktionsgemisch löslichen Katalysatoren, oder heterogen, d.h. unter Verwendung von im Reaktionsgemisch unlöslichen Katalysatoren durchgeführt werden. Der Nachteil der homogenen Verfahren besteht darin, dass der Katalysator den Reaktor mit den Reaktionsprodukten und nicht umgesetzten Edukten verlässt, von denen er abgetrennt, aufgearbeitet und entsorgt oder zurückgeführt werden muss.

Wegen dieses hohen Trennaufwands wird die partielle Oligomerisierung des Isobutens bevorzugt an festen heterogenen Katalysatoren, die zudem oft im Festbett angeordnet sind, durchgeführt, so dass eine aufwändige Katalysatorabtrennung entfällt.

Als Festkatalysatoren können saure Stoffe eingesetzt werden, die im Edukt/Produktgemisch unlöslich sind. Die meisten dieser Katalysatoren gehören einer der folgenden Gruppen an:
a) Mineralsäuren (z. B. Schwefelsäure oder Phosphorsäure) auf einem Trägermaterial (z. B. Aluminiumoxid oder Siliciumdioxid)
b) Zeolithe oder andere Alumosilikate mit oder ohne Dotierung weiterer Metalle, insbesondere mit Übergangsmetallen
c) Saure Ionenaustauscherharze

Wegen der höheren Selektivität für die Bildung von Isobutenoligomeren und wegen der geringeren Bildung von Nebenprodukten werden bevorzugt saure Ionenaustauscherharze als Katalysator verwendet.

Geeignete Ionenaustauscherharze sind beispielsweise solche, die durch Sulfonierung von Phenol/Aldehyd-Kondensaten oder von Cooligomeren von aromatischen Vinylverbindungen hergestellt werden. Beispiele für aromatische Vinylverbindungen zur Herstellung der Cooligomere sind: Styrol, Vinyltoluol, Vinylnaphthalin, Vinylethylbenzol, Methylstyrol, Vinylchlorbenzol, Vinylxylol und Divinylbenzol. Insbesondere werden die Cooligomeren, die durch Umsetzung von Styrol mit Divinylbenzol entstehen, als Vorstufe für die Herstellung von Ionenaustauscherharzen mit Sulfongruppen verwendet. Die Eigenschaften dieser Harze, insbesondere spezifische Oberfläche, Porosität, Stabilität, Quellung bzw. Schrumpfung und Austauschkapazität, können durch den Herstellprozess variiert werden. Die Harze können gelförmig, makroporös oder schwammförmig hergestellt werden. Stark saure Harze des Styrol-Divinylbenzol-Typs werden u. a. unter folgenden Handelsnamen verkauft: CT 151 der Firma Purolite, Amberlyst 15, Amberlyst 35, Amberlite IR-120, Amberlite 200 der Firma Rohm&Haas, Dowex M-31 der Firma DOW, K 2611, K 2431 der Firma Bayer.

Die Ionenaustauscherkapazität der vollständig in der H⁺-Form vorliegenden Harze liegt typischer Weise zwischen 1 bis 2, insbesondere 1,5 bis 1,9 Mol H⁺ pro Liter feuchtes Harz (handelsüblich).

Im Verfahren der Erfindung werden vorzugsweise makroporöse Harze eingesetzt, wie beispielsweise K 2431 der Firma Bayer, Amberlyst 15 oder Amberlyst 35 der Firma Rohm & Haas. Das Porenvolumen beträgt bevorzugt 30 bis 60 ml/g, insbesondere 40 bis 50 ml/g (bezogen auf handelsübliches wasserfeuchtes Harz).

Die Korngröße des Harzes liegt bevorzugt zwischen 500 µm und 1500 µm, insbesondere zwischen 600 µm und 1000 µm.

Die Korngrößenverteilung kann enger oder weiter gewählt werden. So können beispielsweise Ionenaustauscherharze mit sehr einheitlicher Korngröße (monodisperse Harze) eingesetzt werden.

Es kann vorteilhaft sein, in Reaktoren, die mit hohen Lineargeschwindigkeiten durchströmt werden, zur Verringerung des Differenzdruckes ein größeres Korn, und in Reaktoren, die mit einer geringen Lineargeschwindigkeit durchströmt werden, zur Erzielung des optimalen Umsatzes ein kleineres Korn einzusetzen.
Optional können die Ionenaustauscherharze als Formkörper, wie beispielsweise Zylinder, Ringe oder Kugeln, eingesetzt werden.

Das saure Ionenaustauscherharz wird zweckmäßig auf eine Aktivität eingestellt, die zwar die Oligomerisierung des Isobutens ermöglicht, jedoch die Cooligomerisierung von Isobuten mit linearen Butenen, die Oligomerisierung der linearen Butene sowie die Isomerisierung der linearen Butene kaum katalysiert Weiterhin wird die Wärmeentwicklung im Reaktor auf einem technisch gut beherrschbaren Wert eingestellt.

Die Einstellung der gewünschten Katalysatoraktivität kann mit Hilfe von Moderatoren geschehen. Diese Stoffe werden zusammen mit dem Edukt über den Katalysator geleitet. Als Moderator können beispielsweise Wasser, Alkohole wie tert.-Butylalkohol (TBA), Methanol, Isononanol oder Ethanol oder Ether wie tert.-Butylmethylether (MTBE) jeweils als Reinstoff oder Gemische eingesetzt werden. Die Oligomerisierung in Stufe a) wird daher bevorzugt in Gegenwart dieser Moderatoren durchgeführt. Es haben sich hier Molverhältnisse von 0,01 bis 5, bevorzugt 0,01 bis 1, insbesondere 0,01 bis 0,7 Mol Moderator pro Mol Isobuten bewährt.

Im erfindungsgemäßen Verfahren ist es zweckmäßig, denjenigen Alkohol, der zur Veretherung des Isobutens im zweiten Reaktionsschritt eingesetzt wird, oder den im zweiten Reaktionsschritt entstehenden Ether, als Moderator einzusetzen, beispielsweise Methanol oder MTBE.

Im erfindungsgemäßen Verfahren werden zur Oligomerisation weiterhin feste sulfonierte Ionenaustauscherharze eingesetzt, die ohne Zusatz von Moderatoren die gewünschte Aktivität aufweisen. Dies sind insbesondere teilneutralisierte Ionenaustauscherharze, bei denen 1 bis 60 %, bevorzugt 1 bis 30 %, ganz besonders bevorzugt 5 bis 15 % der aciden Protonen der Sulfonsäuregruppen gegen Metallionen ausgetauscht worden sind. Als Metallionen, die die Protonen ersetzen, können Alkalimetall-, Erdalkalimetall-, Übergangsmetallionen (Gruppen 1 - 12 Metallionen wie Chrom-, Mangan-, Eisen-, Kobalt-, Nickel-, Zinkionen) und Aluminiumionen sowie Ionen der Lanthanidgruppe (Seltene Erden) verwendet werden. Bevorzugt werden dafür Alkalimetallionen, insbesondere Natriumionen eingesetzt. Es ist auch möglich, dass das Ionenaustauscherharz mit zwei oder mehreren unterschiedlichen Metallionen beladen ist.

Für die Herstellung der teilneutralisierten Ionenaustauscherharze können verschiedene Verfahren, die alle in der Fachliteratur beschrieben werden, angewendet werden. Liegt das Ionenaustauscherharz in der H⁺-Form vor, können Protonen gegen Metallionen ausgetauscht werden. Liegt das Harz als Metallsalz vor, können Metallionen mit Hilfe von Säuren durch Protonen ersetzt werden. Prinzipiell kann dieser Ionenaustausch sowohl in organischer als in wässriger Suspension erfolgen.

In einem einfachen Verfahren wird beispielsweise das Ionentauscherharz in der H⁺-Form mit so viel Flüssigkeit aufgeschlämmt, dass eine gut rührbare Suspension entsteht. Dazu wird eine Lösung, die die gewünschten Ionen enthält, zudosiert. Nach erfolgten Ionenaustausch wird das teilausgetauschte Ionenaustauscherharz gewaschen und getrocknet.

Die Lösungsmittelmenge zum Aufschlämmen des Ionenaustauscherharz liegt typischerweise beim ein- bis zehnfachen des Eigenvolumens des Ionenaustauscherharzes. Für die Herstellung der Lösung mit der gewünschten Ionenart, die zudosiert wird, ist es ratsam, ein Lösungsmittel zu wählen, das mit dem Lösungsmittel, in dem das Harz suspendiert ist, mischbar ist. Es ist zweckmäßig, gleiche Lösungsmittel zu verwenden.

Der Ionenaustausch erfolgt vorzugsweise im Temperaturbereich von 10 bis 100 °C, besonders bevorzugt bei 20 bis 40 °C. Der Austausch ist in der Regel nach spätestens 24 Stunden abgeschlossen. Nach dem Ionenaustausch wird der Katalysator von der Lösung getrennt, z. B. durch Dekantieren oder Filtrieren, und optional anschließend mit einem Lösungsmittel gewaschen. Es ist zweckmäßig das gleiche Lösungsmittel zu verwenden, in dem der Katalysator suspendiert war.

Es ist vorteilhaft, den feuchten Katalysator zu trocknen, einerseits um ihn handhabbarer (rieselfähiger) zu machen und um andererseits in den ersten Tagen nach dem Anfahren des Reaktors die Verunreinigung des Produktes durch das anhaftende Lösungsmittel oder dessen Folgeprodukte gering zu halten. Die Trocknung kann im Vakuum oder im Inertgasstrom, beispielsweise im Stickstoffstrom, erfolgen. Die Trockentemperaturen liegen typischerweise zwischen 10 und 120 °C.

Ein bevorzugter Weg zur Herstellung der im erfindungsgemäßen Verfahren verwendeten Katalysatoren ist der Austausch von Protonen gegen Metallionen in wässriger Phase, Waschen des teilausgetauschten Ionentauscherharzes mit Wasser und anschließende Trocknung

Die Ionen, mit denen das Harz beladen werden soll, können als Lösungen von Hydroxiden, oder Salzen organischer oder anorganischer Säuren vorliegen. Bei Salzen mehrbasiger Säuren können auch saure Salze eingesetzt werden. Ebenfalls können Verbindungen mit anderen organischen Resten wie beispielsweise Alkoholate oder Acetylacetonate verwendet werden.
Als Quelle für die Metallionen werden bevorzugt Metallhydroxide und Salze anorganischer Säuren eingesetzt. Ganz besonders bevorzugt ist der Einsatz der Alkalimetallhydroxide (zum Beispiel Natriumhydroxid), Alkalimetallhalogenide (zum Beispiel Natriumchlorid), Alkalimetallsulfate (zum Beispiel Natriumsulfat), Alkalimetallnitrate (zum Beispiel Natriumnitrat), Erdalkalimetallhydroxide und Erdalkalimetallnitrate.
Nach dem oben beschriebenen Vorgehen können je nach Austauschgrad, Ionenart und Harz Katalysatoren unterschiedlicher Aktivität und Selektivität hergestellt werden.

Ein Reaktor in dem erfindungsgemäßen Verfahren kann ein Gemisch von Ionenaustauscherharzen unterschiedlicher Reaktivität enthalten. Ebenso ist es möglich, dass ein Reaktor Katalysatoren mit unterschiedlicher Aktivität, in Schichten angeordnet, enthält. Wird mehr als ein Reaktor verwendet, können die einzelnen Reaktoren mit Katalysatoren gleicher oder unterschiedlicher Aktivität gefüllt sein.

Für die technische Ausführung der Umsetzung der Isobuten-haltigen Kohlenwasserstoffmischungen sind diverse Varianten möglich. Die Umsetzung kann chargenweise oder bevorzugt in kontinuierlich arbeitenden Reaktoren, die üblicherweise bei Feststoff/Flüssigkeits-Kontaktreaktionen zum Einsatz gelangen, durchgeführt werden. Bei der Verwendung von kontinuierlich arbeitenden Strömungsreaktoren bedient man sich meistens, jedoch nicht ausschließlich, eines Festbetts. Ein anderes Konzept als Festbettreaktoren sind beispielsweise Reaktoren, in denen der Ionentauscher suspendiert in einer flüssigen Phase vorliegt (vergleiche "Bayer Verfahren", Erdöl und Kohle, Erdgas, Petrochemie, 1974,27, Heft 5, Seite 240).

Wenn ein Festbett-Strömungsreaktor verwendet wird, kann die Flüssigkeit aufwärts oder abwärts strömen. Meistens wird ein Abwärtsströmen der Flüssigkeit bevorzugt. Eine den Reaktor umströmende Kühlflüssigkeit kann gegebenenfalls gleich oder entgegengesetzte Strömungsrichtung aufweisen. Weiterhin ist es möglich, den Reaktor unter Produktrückführung oder im geraden Durchgang zu betreiben.

Bei der Verwendung von Rohrreaktoren kann das Verhältnis von Länge zu Durchmesser der Katalysatorschüttung variiert werden, entweder durch die geometrischen Maße des Reaktors oder durch dessen Füllgrad. Bei gleicher Kontaktmenge und Belastung (LHSV) können somit unterschiedliche Leerrohrgeschwindigkeiten erreicht werden.

Die im technischen Prozess eingesetzten Reaktoren können adiabatisch, polytrop oder praktisch isotherm betrieben werden. Praktisch isotherm bedeutet, dass die Temperatur an einer beliebigen Stelle im Reaktor maximal um 10 °C höher ist als die Temperatur am Reaktoreingang. Bei adiabatischem Betrieb der Reaktoren ist es in der Regel sinnvoll, mehrere Reaktoren in Reihe zu schalten und zwischen den Reaktoren zu kühlen. Reaktoren, die für einen polytropen oder praktisch isothermen Betrieb geeignet sind, sind beispielsweise Rohrbündelreaktoren, Rührkessel und Schlaufenreaktoren.

Es ist möglich, mehrere Reaktoren, auch verschiedene Bauarten, zu kombinieren. Es ist zudem möglich, Reaktoren unter Rückführung von Produkt zu betreiben.

Die Temperaturen, bei denen die Oligomerisation betrieben wird, liegen zwischen 5 und 160 °C, vorzugsweise zwischen 40 und 110 °C.

Die Umsetzung kann mit und ohne Zugabe eines zusätzlichen Lösungsmittels erfolgen. Als Lösungsmittel werden bevorzugt gesättigte Kohlenwasserstoffe eingesetzt, insbesondere C₄-, C₈- oder C₁₂ Kohlenwasserstoffe. Ganz besonders bevorzugt ist der Einsatz von Isooktan. Bei Zugabe von Lösungsmitteln beträgt ihr Anteil 0 bis 60 Gew.%, bevorzugt 0 bis 30 Gew.%.

Die erfindungsgemäße Umsetzung kann bei einem Druck gleich oder über dem Dampfdruck des Einsatz-Kohlenwasserstoffgemisches bei der jeweiligen Reaktionstemperatur durchgeführt werden, vorzugsweise bei einem Druck unter 40 bar, d. h. die Isobuten-haltigen Kohlenwasserstoffmischungen liegen während der Oligomerisation ganz oder teilweise in flüssiger Phase vor. Wenn die Reaktion vollständig in der Flüssigphase durchgeführt werden soll, sollte der Druck 2 bis 4 bar höher als der Dampfdruck des Reaktionsgemisches sein, um in den Reaktoren Verdampfungsprobleme zu vermeiden.

Auch wenn die Reaktion bei einem Druck betrieben wird, bei dem die Reaktionsmischung nicht vollständig flüssig vorliegt (beispielsweise in einer Reaktivdestillation oder bei Verfahrensvarianten analog US 5 003 124), findet die Oligomerisierung nach dem erfindungsgemäßen Verfahren trotzdem in der Flüssigphase, also an "feuchtem" d. h. mit Flüssigkeit benetztem Katalysator statt.

Der Gesamtumsatz an Isobuten zu Oligomeren kann über die Art und Menge des verwendeten Katalysators, den eingestellten Reaktionsbedingungen und Anzahl der Reaktoren eingestellt werden. Im erfindungsgemäßen Verfahren werden 50 bis 95 % des im Edukt enthaltenden Isobutens, insbesondere 60 bis 90 %, oligomerisiert.

Das Reaktionsgemisch der partiellen Isobuten-Oligomerisierung kann unterschiedlich aufgearbeitet werden. Das Gemisch wird entweder direkt in die Veretherung geleitet oder vor der Veretherung werden die Butenoligomeren abgetrennt. Die Abtrennung der Oligomeren und gegebenenfalls von Kohlenwasserstoffen mit 5 bis 7 Kohlenstoffatomen erfolgt zweckmäßig durch Destillation.

Die abgetrennte Oligomerfraktion enthält hauptsächlich C₈-Kohlenwasserstoffe. Diese kann neben dem Diisobuten auch Codimere und höhere Oligomere (C₁₂, C₁₆,) enthalten. Diese Fraktion kann in weiteren Destillationsschritten aufgetrennt werden. So ist es beispielsweise möglich, eine Fraktion mit hochreinem Diisobuten abzutrennen, um diese separat, beispielsweise für chemische Synthesen, einzusetzen. Für den Einsatz als Kraftstoflkomponente für Ottomotoren kann es notwendig sein, hochsiedende Komponenten (vorzugsweise Siedepunkt >220 °C) abzutrennen.

Es ist auch möglich die Butenoligomeren, insbesondere die C₈-Olefine, ganz oder teilweise zu hydrieren. Methoden zur Hydrierung der Produkte der Oligomerisierung zu den entsprechenden Paraffinen sind dem Fachmann hinlänglich bekannt. Gängige Methoden zur Hydrierung von Olefinen sind beispielsweise beschrieben in F. Asinger, "Chemie und Technologie der Monoolefine", Akademie Verlag, Berlin, 1957, Seite 626 - 628 oder DE 197 19 833.

In einer bevorzugten Ausführungsform wird die Hydrierung in flüssiger Phase an einem festen, im Hydriergut nicht löslichen Katalysator durchgeführt. Als Hydrierkatalysatoren werden bevorzugt Trägerkatalysatoren, die aus einem anorganischen Träger bestehen und als Aktivmetall Platin und/oder Palladium und/oder Nickel enthalten, verwendet. Die Temperatur, bei der die Hydrierung durchgeführt wird, liegt bevorzugt im Bereich von 10 bis 250 °C und der Druck zwischen 1 und 100 bar.

Nach der Hydrierung können durch destillative Trennung weitere Fraktionen gewonnen werden. Aus diesen und aus den unhydrierten Fraktionen sind durch Abmischung Kraftstoffadditive bestimmter Eigenschaften erhältlich. Weiterhin können einige Fraktionen als Lösemittel verwendet werden.

Das an Isobuten verarmte C₄-Kohlenwasserstoffgemisch wird im erfindungsgemäßen Verfahren in einem zweiten Reaktionsschritt (Stufe b) umgesetzt, in der das restliche Isobuten durch Anlagerung von Alkohol zum entsprechenden tertiären Ether entfernt wird Diesem C₄-Kohlenwasserstoffgemisch können bei Bedarf noch zusätzliche isobutenarme C₄-Kohlenwasserstoffe (beispielsweise Raffinat II, ggf. zugekauft) zugemischt werden. Diese Ströme können aus anderen Prozessen stammen, beispielsweise einer TBA Synthese oder einer Isobuten-Abtrennung über Hydroisomerisierung/Destillation (vgl. EP 1 184 361).

Die Veretherung des Isobutens wird als sauer katalysierte Reaktion durchgeführt. Als Alkohole werden dafür primäre, sekundäre, ein- oder mehrwertige Alkohole mit 1 bis 5 C-Atomen, bevorzugt Methanol oder Ethanol eingesetzt.

Um einen nahezu vollständigen Umsatz des Rest-Isobutens zu erreichen, wird die Addition des Alkohols an das Isobuten in Gegenwart eines sauren Katalysators in mindestens zwei Reaktionsstufen durchgeführt, wobei die letzte Reaktionsstufe als Reaktivdestillation durchgeführt wird. In dem/den Vorreaktor/en wird zunächst an einem sauren Katalysator aus dem Isobuten armen C₄-Strom und Alkohol ein Reaktionsgemisch hergestellt, das hinsichtlich seiner Isobuten-, Alkohol- und tert.-Butylether-Konzentration in der Nähe des thermodynamischen Gleichgewichts liegt. Dies liegt beispielsweise für Methanol /MTBE in der Regel zwischen 94 und 96 % Isobuten-Umsatz. Dieses Gemisch wird in die Reaktivdestillationskolonne eingespeist, wo ein weiterer Teil des Isobutens zum Ether umgesetzt wird.

Der oder die Vorreaktoren, in denen der Alkohol mit dem Isobuten bis nahe an das thermodynamische Gleichgewicht umgesetzt werden, können herkömmliche Festbettreaktoren (Rohrbündelreaktoren, Kreislaufreaktoren) sein. Sie können mit oder ohne partieller Rückführung, wobei ggf der Rückführungsstrom gekühlt wird, betrieben werden.

Die Reaktoren werden in üblicher Weise bei 30 bis 110 °C und 5 bis 50 bar_{abs}. (bara) betrieben. Da das thermodynamische Gleichgewicht zwischen Alkohol/Isobuten und Ether bei tiefer Temperatur überwiegend auf der Seite des Ethers liegt, ist es bei Einsatz mehrerer Vorreaktoren bevorzugt, den ersten der Reaktoren bei höherer Temperatur (hohe Reaktionsgeschwindigkeit) als die Folgenden (Ausnutzung der Gleichgewichtslage) zu betreiben.

Als Katalysator wird sowohl im Vorreaktor als auch in der Reaktivdestillationskolonne ein fester Stoff, der weder im Einsatzstoffgemisch noch im Produktgemisch löslich ist, mit sauren Zentren an seiner Oberfläche eingesetzt. Der Katalysator darf unter Reaktionsbedingungen keine sauren Stoffe an das Produktgemisch abgeben, weil dies zu Ausbeuteverluste führen würde.

In der Reaktivdestillationskolonne ist der Katalysator entweder in der Packung integriert, beispielsweise KataMax^{®} (EP 0 428 265), KataPak^{®} (EP 0 396 650) oder MultiPak^{®} (Gebrauchsmuster Nr. 298 07 007.3) oder auf Formkörpern aufpolymerisiert (US 5 244 929).

Für die Aktivität der Katalysatoren gilt, dass sie unter Reaktionsbedingungen die Addition von Alkohol an Isobuten bewirken, jedoch kaum die Addition an lineare Butene. Weiterhin dürfen sie die Oligomerisierung von linearen Butenen und die Dialkyletherbildung aus zwei Molekülen eingesetzten Alkohols kaum katalysieren. Im Hinblick auf eine hohe Ausbeute an 1-Buten sollte die Aktivität für die Gleichgewichtseinstellung zwischen den linearen Butenen gering sein.

Als feste Katalysatoren können beispielsweise Zeolithe, säureaktivierte Betonite und/oder Tonerden, sulfonierte Zirkoniumoxide, Monomorillonite oder saure Ionenaustauscherharze verwendet werden.

Eine im erfindungsgemäßen Verfahren bevorzugte Gruppe von sauren Katalysatoren sind feste Ionenaustauscherharze mit Sulfonsäuregruppen. Geeignete Ionenaustauscherharze sind beispielsweise solche, die durch Sulfonierung von Phenol/Aldehyd-Kondensaten oder von Cooligomeren von aromatischen Vinylverbindungen hergestellt werden. Beispiele für aromatische Vinylverbindungen zur Herstellung der Cooligomere sind: Styrol, Vinyltoluol, Vinylnaphthalin, Vinylethylbenzol, Methylstyrol, Vinylchlorbenzol, Vinylxylol und Divinylbenzol. Insbesondere werden die Cooligomeren, die durch Umsetzung von Styrol mit Divinylbenzol entstehen, als Vorstufe für die Herstellung von Ionenaustauscherharzen mit Sulfonsäuregruppen verwendet. Die Harze können gelförmig, makroporös oder schwammförmig hergestellt werden. Stark saure Harze des Styrol-Divinylbenzol-Typs werden u. a. unter folgenden Handelsnamen verkauft: Duolite C20, Duolite C26, Amberlyst A15, Amberlyst A35, Amberlite IR-120, Amberlite 200, Dowex 50, Lewatit SPC 118, Lewatit SPC 108, K2611, K2621, OC 1501.

Die Eigenschaften dieser Harze, insbesondere spezifische Oberfläche, Porosität, Stabilität, Quellung bzw. Schrumpfung und Austauschkapazität, können durch den Herstellprozess variiert werden.

Im erfindungsgemäßen Verfahren können die Ionenaustauscherharze in ihrer H-Form eingesetzt werden. Es werden vorzugsweise makroporöse Harze eingesetzt, wie beispielsweise Lewatit SCP 118, Lewatit SCP 108, Amberlyst A15 oder Amberlyst A35, K2621. Das Porenvolumen beträgt 0,3 bis 0,9 ml/g , insbesondere 0,5 bis 0,9 ml/g. Die Korngröße des Harzes liegt zwischen 0,3 mm und 1,5 mm, insbesondere zwischen 0,5 mm und 1,0 mm. Die Korngrößenverteilung kann enger oder weiter gewählt werden. So können beispielsweise Ionenaustauscherharze mit sehr einheitlicher Korngröße (monodisperse Harze) eingesetzt werden. Die Kapazität des Ionenaustauscher beträgt, bezogen auf Lieferform, 0,7 - 2,0 mol/l, insbesondere 1,1-2,0 mol/l.

Die Umsetzung des Rest-Isobutens mit Alkohol zum entsprechenden tertiären Butylether erfolgt im Temperaturbereich von 10 - 140 °C. Dabei wird der Alkohol im Überschuss eingesetzt.

Insbesondere wird das Rest-Isobuten durch Umsetzung mit Methanol zu MTBE entfernt. Dabei wird insbesondere derart verfahren, wie in DE 101 02 082 beschrieben. Das Isobuten arme C₄-Kohlenwasserstoffgemisch wird zusammen mit Methanol in den Vorreaktor eingespeist. Dort entsteht ein Gemisch, in dem Isobuten, Methanol und MTBE im Gleichgewicht oder nahezu im Gleichgewicht stehen. Dieses Reaktionsgemisch wird in die Reaktivdestillationskolonne eingeleitet.

Im Zulauf der Reaktivdestillationskolonne kann mehr Methanol enthalten sein, als für die vollständige Umsetzung des noch vorhandenen Isobutens gebraucht wird. Der Methanolüberschuss sollte jedoch derart begrenzt werden, dass einerseits eine ausreichende Methanolmenge für das sich bildende Azeotrop aus Methanol und C₄-Kohlenwasserstoffen vorhanden ist, andererseits nicht so viel, dass Methanol ins Sumpfprodukt geriete, so dass ein spezifikationsgerechtes MTBE (Methanolgehalt bevorzugt unter 5000 wppm) erhalten wird. Dies gilt allerdings nur dann, wenn vorher die Oligomeren abgetrennt wurden. Ansonsten wird ein Gemisch aus MTBE und Oligomeren erhalten.

Optional kann, wenn der Methanolgehalt im Kolonnenzulauf unter dem maximal zulässigen Wert liegt, zusätzliches Methanol zugemischt werden. Darüber hinaus kann am Kopf der Reaktivdestillationskolonne über eine gesonderte Einrichtung eine Methanol-Einspeisung erfolgen.

In der Reaktivdestillationskolonne besteht die Zone oberhalb der Katalysatorpackung aus 5 bis 20, insbesondere aus 10 bis 15 Trennstufen. Die Katalysatorzone lässt sich mit einer destillativen Wirkung von 1 bis 5 theoretischen Trennstufen pro Meter Packungshöhe abschätzen. Die Trennzone unterhalb des Katalysators umfasst 12 bis 36, insbesondere 20 bis 30 Trennstufen.

Die Temperatur des Kolonnenzulaufs liegt unabhängig von dessen Zusammensetzung, Reaktionsdruck in der Kolonne und Durchsatz zwischen 50 °C und 80 °C, vorzugsweise zwischen 60 °C und 75 °C.

Die mittlere Temperatur in der Katalysatorzone beträgt abhängig vom Druck in der Kolonne vorzugsweise 55 °C bis 70 °C, besonders bevorzugt 58 °C bis 67 °C.

Die Reaktivdestillationskolonne wird bei Drücken, gemessen am Kolonnenkopf, von 3 bis 15, bevorzugt 5 bara bis 9 bara betrieben, insbesondere von 7 bara bis 8,5 bara.
Die hydraulische Belastung in der katalytischen Packung der Kolonne beträgt bevorzugt 10 % bis 110 %, vorzugsweise 20 % bis 70 % ihrer Flutpunktbelastung. Unter hydraulischer Belastung einer Destillationskolonne wird die gleichmäßige strömungstechnische Beanspruchung des Kolonnenquerschnitts durch den aufsteigenden Dampf-Massenstrom und den rücklaufenden Flüssigkeits-Massenstrom verstanden. Die obere Belastungsgrenze kennzeichnet die maximale Belastung durch Dampf und Rücklaufflüssigkeit, oberhalb derer die Trennwirkung infolge Mitreißens oder Stauens der Rücklaufflüssigkeit durch den aufsteigenden Dampfstrom absinkt. Die untere Belastungssgrenze kennzeichnet die minimale Belastung, unterhalb derer die Trennwirkung absinkt oder zusammenbricht infolge unregelmäßiger Strömung oder Leerlaufen der Kolonne - z. B. der Böden. (Vauck/Müller, "Grundoperationen chemischer Verfahrenstechnik", S. 626, VEB Deutscher Verlag für Grundstoffindustrie.)

Am Flutpunkt werden die vom Gas an die Flüssigkeit übertragenen Schubspannungen so groß, dass die gesamte Flüssigkeitsmenge in Form von Tropfen mit dem Gas mitgerissen wird oder dass es zu Phaseninversion in der Kolonne kommt (J. Mackowiak, "Fluiddynamik von Kolonnen mit modernen Füllkörpern und Packungen für Gas/Flüssigkeitssysteme", Otto Salle Verlag 1991).

Die Reaktivdestillationskolonne wird mit Rücklaufverhältnissen kleiner als 1,5 betrieben, insbesondere mit solchen, die größer 0,4 und kleiner 1, bevorzugt zwischen 0,5 und 0,9 liegen.

Das optimale Rücklaufverhältnis hängt von dem Durchsatz der Zusammensetzung des Kolonnenzulaufs und dem Kolonnendruck ab. Es liegt jedoch immer in den oben genannten Bereichen. Im Destillat der Reaktivdestillation werden Isobuten-Restkonzentrationen im Raffinat II (gängige Bezeichnung von 1,3-Butadien und Isobuten befreite Mischung von C₄-Kohlenwasserstoffen) von kleiner 1000, bevorzugt 500, ganz besonders bevorzugt kleiner 300 wppm (bezogen auf das C₄-Gemisch im Destillat) erhalten.

Werden die Butenoligomeren vor der Umsetzung mit Methanol abgetrennt, besteht das Sumpfprodukt der Reaktivdestillationskolonne bevorzugt aus MTBE. Bevorzugt enthält es weniger als 2500 wppm Methyl-sec.-butylether und weniger als 2500 wppm C₈-Kohlenwasserstoffe.

Das Kopfprodukt der Reaktivdestillation kann wiederum in ein C₄-Kohlenwasserstoffgemisch und Methanol getrennt werden, wobei das C₄-Kohlenwasserstoffgemisch bevorzugt weniger als 0,5 wppm MTBE und/oder TBA enthält.

Das Methanol kann z. B. durch Extraktion mit Wasser abgetrennt werden. Falls Spuren von Butadien nicht schon vor der Butenoligomerisation entfernt wurden, können sie aus dem so erhaltenen Raffinat II durch Selektivhydrierung (SHP) entfernt werden. Das aus der Reaktivdestillation erhaltene und ggf. von Methanol befreite C₄-Kohlenwasserstoffgemisch kann destillativ weiter aufgetrennt werden. Dazu bieten sich zwei bevorzugte Routen an:
1. Trennung in eine Kopffraktion, die 1-Buten und Isobutan enthält, und eine Sumpffraktion, die n-Butan, 2-Butene und ggf. Restmengen an 1-Buten enthält. Aus der Kopffraktion kann in einer weiteren Destillation das Isobutan abgetrennt werden, so dass hochreines 1-Buten zurückbleibt oder
2. Abtrennung des Isobutans und ggf. weiterer Leichtsieder in einem ersten Destillationsschritt, wobei im Sumpf 1-Buten, 2-Butene und n-Butan zurückbleiben. Aus dem Sumpfprodukt kann in einer weiteren Destillation 1-Buten als Leichtsieder gewonnen werden.

Werden für die Veretherung andere Alkohole als Methanol eingesetzt, so ändern sich die Parameter der Reaktivdestillation entsprechend.

Das derart hergestellte, reine 1-Buten enthält weniger als 1000 wppm Isobuten und ist ein gefragtes Zwischenprodukt. Es wird beispielsweise als Comonomer bei der Herstellung von Polyethylen (LLDPE oder HDPE) sowie von Ethylen-Propylen-Mischpolymeren eingesetzt. Es findet weiterhin Einsatz als Alkylierungsmittel und ist Ausgangsstoff für die Herstellung von Butan-2-ol, Butenoxid, Valeraldehyd.

Eine weitere Verwendung des erfindungsgemäß hergestellten nahezu Isobuten-freien, Raffinats II ist die Herstellung von n-Buten-Oligomeren, insbesondere nach dem Octol-Prozess.
Die nach Abtrennung bzw. Umsetzung der linearen Butene aus dem Raffinat II zurückbleibenden Kohlenwasserstoffe können gegebenenfalls nach Hydrierung (CSP Complete saturation process) zu Isobutan und n-Butan aufgearbeitet werden.

Der in der Reaktivdestillation als Sumpfprodukt anfallende tert.-Butylether kann for verschiedene Zwecke genutzt werden. Neben dem Einsatz als Komponente für Ottokraftstoffe findet er beispielsweise Verwendung als Lösungsmittel. Durch Rückspaltung der tert.-Butylether ist Isobuten hoher Reinheit erhältlich.

Der bei Verwendung von Methanol anfallende MTBE wird beispielsweise, neben der Verwendung als Komponente im Ottokraftstoff, als Lösungsmittel genutzt. Zur Gewinnung von MTBE hoher Reinheit, welcher bevorzugt als Lösungsmittel eingesetzt wird, kann das im Prozess anfallende Sumpfprodukt der Reaktivdestillation destillativ weiter aufgereinigt werden. Dabei wird der Gehalt an in geringer Menge enthaltenen Verunreinigungen (beispielsweise Methyl-sec.-butylether, C₈-KW, TBA, Alkohole) reduziert.

Die Rückspaltung von MTBE zur Gewinnung von Isobuten ist beispielsweise in DE 100 20 943 beschrieben. Die dabei erhaltene Reinheit des Isobutens ist unter anderem abhängig vom Anteil an Methyl-sec.-butylether im MTBE. Je nach Anforderungen wird daher ein unterschiedlich intensiv vorgereinigtes MTBE für die Rückspaltung eingesetzt.

Werden vor der Veretherung die Oligomeren nicht abgetrennt, fällt in der Reaktivdestillationskolonne ein Sumpfprodukt an, das MTBE und die Oligomeren enthält. Dieses Gemisch kann als solches oder nach Hydrierung der olefinischen Doppelbindungen als Kraftstoftkomponente verwendet werden. Optional kann dieses Gemisch fraktioniert werden. Die einzelnen Fraktionen werden dann genutzt, wie bereits beschrieben.

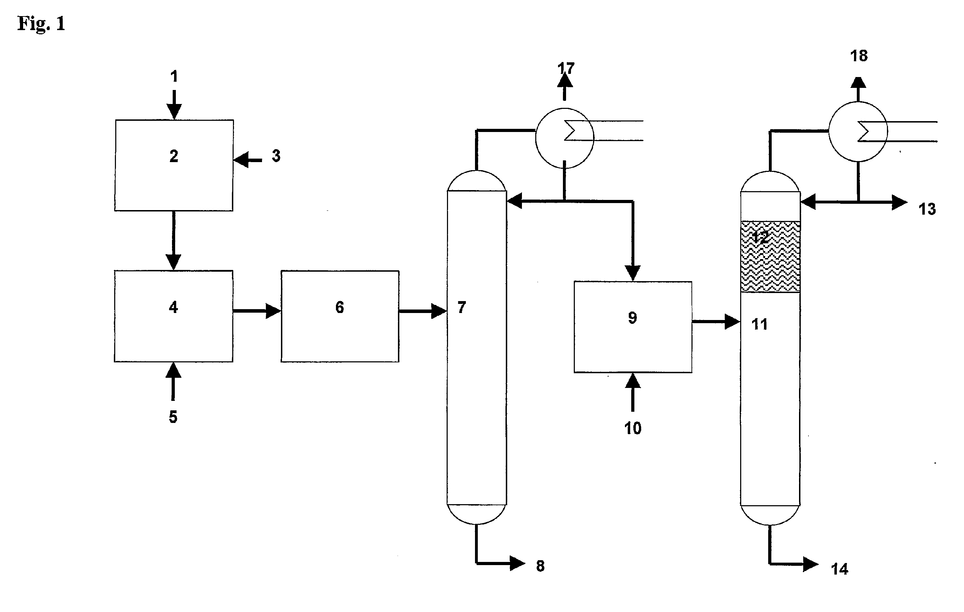

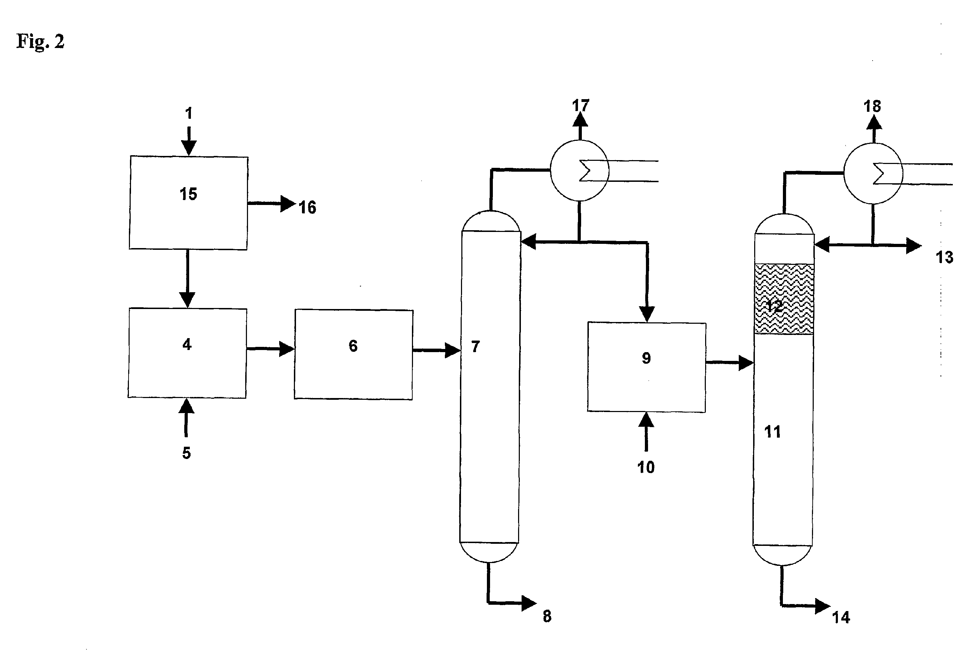

Zwei Ausführungsformen des erfindungsgemäßen Verfahrens werden an Hand der Figuren Fig. 1 und Fig. 2 schematisch näher erläutert, ohne dass die Erfindung auf diese Ausführungsarten beschränkt sein soll. In den schematischen Darstellungen sind nur die wesentlichen Stufen dargestellt. Auf die Darstellung von verfahrenstechnisch üblichen Strömen, wie z. B. Kühlwasserströmen, Kreislaufströmen, Katalysatorrückführungen oder Rückspeisungen, und/oder üblichen Apparaturen, wie z. B. Wärmetauschern oder Abscheidern wurde zu Gunsten einer besseren Übersicht verzichtet.
Bei dem in Fig.1 schematisch dargestellten Verfahren wird ein Isobuten- und 1,3-Butadienhaltiger C₄-Kohlenwasserstoffstrom **1** in einen oder mehrere Reaktoren, in welchem eine Butadien-Hydrierung durchgeführt wird, **2** eingespeist. In diesen Reaktor wird zur Hydrierung außerdem Wasserstoff **3** eingespeist. Der Reaktoraustrag wird in einen nächsten Reaktor zur Rest-Butadien Selektiv-Hydrierung **4** überführt, in welchen wiederum Wasserstoff **5** eingespeist wird. Der Reaktoraustrag aus **4** wird in den Reaktor zur Buten-Oligomerisierung **6** überführt, in dem hauptsächlich das Isobuten zu Dimeren, Trimeren und Tetrameren umgesetzt wird. Der Austrag aus diesem Reaktor wird in eine Destillationskolonne **7** überführt, in welcher die Buten-Oligomeren als Sumpfprodukt **8** abgetrennt werden. Über den Kopf der Kolonne **7** wird der von Buten-Oligomeren befreite Stoffstrom in einen Wärmetauscher gefahren, in welchem die kondensierbaren Bestandteile kondensiert werden. Teilweise werden diese als Rücklauf in den Kopf der Kolonne **7** zurückgefahren. Im Wärmetauscher nicht kondensierbare Bestandteile werden als gasförmiger Abgasstrom **17** einer weiteren Verwertung zugeführt. Das Destillat der Kolonne **7** wird zusammen mit zusätzlichem Methanol **10** in die MTBE-Festbettstufe **9** eingespeist, mit welchem das noch vorhandene Isobuten an dem Festbettkatalysator zu MTBE umgesetzt wird. Der Austrag aus der Festbettstufe wird unterhalb der reaktiven Packung **12** in eine Reaktivdestillationskolonne **11** eingespeist. Am Sumpf der Kolonne wird ein MTBE **14** aufweisender Strom angenommen, während das Kopfprodukt in einen Wärmetauscher gefahren wird, in welchem die kondensierbaren Bestandteile als Isobuten-freier n-Buten-Strom kondensiert werden. Diese werden teilweise als Rücklauf in den Kopf der Kolonne **11** zurückgefahren und als Strom **13** aus dem Prozess ausgeschleust. Die im Wärmetauscher nicht kondensierbaren Anteile des Kopfproduktes werden als gasförmiger Abgasstrom **18** einer weiteren Verwertung zugeführt.

Das in Fig. 2 schematisch dargestellte Verfahren unterscheidet sich von dem in Fig. 1 dargestellten Verfahren dadurch, dass statt der Butadienhydrierung **2** eine Butadienabtrennung **15,** beispielsweise eine Extraktivdestillation, durchgeführt wird, durch welche aus dem Isobuten-haltigen C₄-Kohlenwasserstoffstrom **1** ein Großteil des 1,3-Butadiens **16** entfernt wird. Der an Butadien abgereicherte Strom aus der Butadienabtrennung **15** wird anschließend in einen Reaktor zur Rest-Butadien Selektiv-Hydrierung **4** überführt, und dann wie in Fig. 1 weiter aufgearbeitet.
Die folgenden Beispiele sollen die Erfindung erläutern, ohne den Schutzbereich der Patentansprüche einzuengen.

### Beispiele

Die einzelnen Verfahrensschritte für die Aufarbeitung eines Crack-C₄ Strom (CC₄) analog zum in Figur 1 dargestellten Verfahren wurden im Labor nachgestellt.

### Beispiel 1 - Butadien-Selektivhydrierung

Ein KW-Gemisch mit der in Tabelle 2 angegebenen Zusammensetzung wird über einem heterogenen Palladiumkatalysator in einem Festbettreaktor hydriert. Die Hydrierung analog zu EP 0 523 482 wird in zwei Festbettreaktoren durchgeführt, die in Serie betrieben werden und jeweils mit einem Abscheider und einem Flüssigkeitskreislauf ausgestattet sind. In jedem der Flüssigkeitskreisläufe ist ein Kühler installiert, um die Reaktionswärme der Hydrierung abführen zu können.

Der für die Reaktion notwenige Wasserstoff wurde den Reaktoren entsprechend der umzusetzenden Menge an 1,3-Butadien zugeführt. Die LHSV über die gesamte Anlage betrug 8.5 t/(m³ h)
Prozentangaben der Analysen sind als Massen-% zu lesen.

| | | |
|---|---|---|
| 1.Stufe | Eintrittstemperatur Reaktor | 29°C |
| | Druck | 14 bara |
| | Katalysator | 0.5 % Pd auf Al₂O₃ |
| 2. Stufe | Eintrittstemperatur Reaktor | 45°C |
| | Druck | 9 bara |
| | Katalysator | 0.5 % Pd auf Al₂O₃ |

**Tabelle 2 - Analysen von Zulauf und Austrag der Butadienhydrierung**

| | **Feed** | **Austrag 1. Stufe** | **Austrag 2. Stufe** |
|---|---|---|---|
| C3-KW [%] | n.o., <0,1 | n.o., <0,1 | n.o., < 0,1 |
| iso-Butan [%] | 2,3 | 2,3 | 2,3 |
| n-Butan [%] | 5,5 | 5,9 | 6,3 |
| trans-Buten [%] | 4,9 | 17,2 | 19,7 |
| 1-Buten [%] | 15,9 | 37,1 | 40,4 |
| iso-Buten [%] | 24,8 | 24,8 | 24,8 |
| cis-Buten [%] | 4,0 | 5,1 | 6,1 |
| 1,2-Butadien [%] | 0,2 | no., < 0,1 | no., < 0,1 |
| 1,3-Butadien [%] | 41,4 | 6,3 | 0,2 |
| Butenin [%] | 0,75 | no., < 0,1 | no., < 0,1 |
| 1-Butin [%] | 0,2 | no., < 0,1 | no., < 0,1 |

| | | | |
|---|---|---|---|
| n.o. = unter der Nachweisgrenze | | | |

### Beispiel 2 -SHP, weitere Absenkung des 1,3-Butadien-Gehalts

Restmengen an 1,3-Butadien, die noch im C₄-Kohlenwasserstoffgemisch enthalten sind, können in einem weiteren Hydrierungsschritt reduziert werden. Durch Zugabe geringer Mengen Kohlenmonoxid wird dabei die Selektivität des Palladiumkatalysators erhöht.

Der Versuch wurde mit zwei unterschiedlichen Rohstoffen durchgeführt. Der unter 2b angegebene Zulauf entspricht einer Zusammensetzung, wie sie aus einem Prozess nach Beispiel 1 erhalten wird (Hydrierung des im Crack-C₄-enthaltenen 1,3-Butadiens). Der Zulauf 2a steht für eine Mischung von C₄-Kohlenwasserstoffen, die nach einer Abtrennung des 1,3-Butadiens aus dem Crack-C₄ erhalten wird (Fig. 2).

Die KW-Gemische mit der in Tabelle 3 angegebenen Zusammensetzung werden mit 85 ppm Wasserstoff und 2 ppm Kohlenmonoxid beaufschlagt. Die Hydrierung erfolgte in einem Festbettreaktor unter folgenden Bedingungen.
Temperatur: 40 °C (isotherm)
Druck: 13 bara
LHSV: 35 (1/(1*h))
Katalysator: 0.5% Pd auf γ-Al₂O₃-Träger

**Tabelle 3 - Analyse von Zulauf und Austrag des SHP (selective hydrogenation process)**

| **Beispiel** | **2a Zulauf** | **2a Austrag** | **2b Zulauf** | **2b Austrag** |
|---|---|---|---|---|
| C3-KW [%] | 0,0 | 0,0 | <0,1 | <0,1 |
| Iso-Butan [%] | 3,9 | 3,9 | 2,3 | 2,3 |
| n-Butan [%] | 9,5 | 9,5 | 5,9 | 5,9 |
| Trans-Buten [%] | 8,5 | 8,6 | 17,6 | 17,6 |
| 1-Buten [%] | 27,6 | 27,7 | 40,9 | 41,1 |
| Iso-Buten [%] | 43,1 | 43,1 | 24,8 | 24,8 |
| Cis-Buten [%] | 6,9 | 6,9 | 8,2 | 8,2 |
| 1,2-Butadien [ppm] | <100 | n.o. | <1 | n.o. |
| 1,3-Butadien [ppm] | 2176 | 3 | 1960 | 2 |
| Butenin [ppm] | n.o. | n.o. | 12 | n.o. |
| 1-Butin [ppm] | n.o. | n.o. | 56 | n.o. |

| | | | | |
|---|---|---|---|---|
| n.o. = unter der Nachweisgrenze | | | | |

### Beispiel 3 - Isobutenoligomerisierung und Destillation

Die Oligomerisierung des Isobuten erfolgte in einem Rohrreaktor von 200 cm Länge (zu einer Spirale gewickelt), Innendurchmesser 6 mm. Das Rohr wird von außen durch ein Ölbad temperiert. Als Katalysator wurden 54 ml Amberlyst 15 der Firma Rohm & Haas eingesetzt. Vor dem Einsatz wurde der Katalysator mit Wasser, anschließend mit Methanol gewaschen. Der Reaktor wurde über eine Druckhaltung am Reaktorausgang bei konstant 22 bara betrieben. Nach Inbetriebnahme wurden die Versuchsanlage 24 Stunden betrieben und das Produkt verworfen. Danach wurde das Produkt der Anlage 100 Stunden gesammelt und in einer Laborkolonne destilliert. Die dabei erhaltenen Fraktionen wurden gaschromatographisch analysiert. In Tabelle 4 sind die Fahrbedingungen, Eduktzusammensetzungen und die Analysen der C₄- und der C₈-Fraktion zusammengestellt. Dabei bedeuten
- Umsatz Isobuten: Anteil an Isobuten, der im Reaktor umgesetzt wurde.
- C₈-Selektivität: Selektivität, mit der bei der Reaktion C₈-Kohlenwasserstoffe (Dimere der Butene) gebildet wurden.
- Der unter C₈-Analyse angegebene Wert für 2,4,4-Trimethlylpenten gibt an, welchen Anteil (Masseprozent) die Dimere des Isobutens in der C₈-Fraktion haben.

Es wurden zwei Versuche (3a, 3b) mit unterschiedlich zusammengesetzten Zuläufen durchgeführt. Bei jedem Versuch wurde frischer Katalysator verwendet.

**Tabelle 4 - Reaktionsbedingungen und Analyse der Isobutenoligomerisierung**

| **Versuch Nr**. | | 3a | 3b |
|---|---|---|---|
| Temp. Wärmebad | (°C) | 62.5 | 62.5 |
| Zulauf Reaktor | (kg/h) | 0.300 | 0.306 |

| **Analyse Zulauf** | | | |
|---|---|---|---|
| Propen | (%) | 0.01 | 0.00 |
| iso-Butan | (%) | 2.22 | 2.07 |
| n-Butan | (%) | 10.32 | 9.20 |
| trans-Buten | (%) | 25.83 | 9.21 |
| 1-Buten | (%) | 2.92 | 27.10 |
| iso-Buten | (%) | 45.84 | 46.12 |
| cis-Buten | (%) | 12.67 | 5.825 |
| Rest | (%) | 0.20 | 0.48 |

| **Austrag Analyse C4-Fraktion (auf 100% C4 normiert)** | | | |
|---|---|---|---|
| iso-Butan | (%) | 4.16 | 3.74 |
| n-Butan | (%) | 17.41 | 15.77 |
| trans-Buten | (%) | 42.41 | 16.84 |
| 1-Buten | (%) | 4.84 | 42.67 |
| iso-Buten | (%) | 11.71 | 9.75 |
| cis-Buten | (%) | 19.21 | 10.66 |
| Rest | (%) | 0.25 | 0.58 |
| | | | |
| **Umsatz Isobuten** | (%) | 84.33 | 87.38 |
| C8-Selektivität | (%) | 77.82 | 77.91 |

| **Analyse der C8-Fraktion (auf 100% C8 normiert)** | | | |
|---|---|---|---|
| 2,4,4-Tri-Me-Penten | (%) | 91.40 | 92.60 |

### Beispiel 4 - Isobutenoligomerisierung am teilneutralisieren Ionentauscher

### a) Herstellung eines teilneutralisierten Katalysators, Einstellung der Säurekapazität

Der eingesetzte Ionenaustauscher der Fa. Rohm und Haas (Amberlyst 15) hatte eine ursprüngliche Säurekapazität von 1,7 mol H+/l. Zur Einstellung der gewünschten Aktivität wurden 40 % der sauren Zentren neutralisiert.

Hierfür wurden 1000 ml des Ionenaustauscherharzes in 1000 ml VE-Wasser aufgeschlämmt und unter Rühren eine Lösung aus 27,2 g Natriumhydroxid (0,68 mol) und 500 ml VE-Wasser in einer Stunde im Temperaturbereich 20 bis 40 °C zugetropft. Es wurde 5 Min nachgerührt und danach das Ionenaustauscherharz mit dreimal 1000 ml VE-Wasser neutral gewaschen. Die anschließende Kapazitätsmessung des teilneutralisierten Ionenaustauschers ergab 1,00 +/- 0,03 mol H+/1. Der Katalysator wurde vor Einsatz 15 h bei 70 °C getrocknet.

### b) Oligomerisierung

Die Oligomerisierung des Isobuten erfolgte analog zu Beispiel 3. Es wurden 51 ml des teilneutralisierten Ionentauschers eingesetzt. Der Reaktor wurde bei 22 bara betrieben. Nach einer Formierungsphase von 24 Stunden wurde der Austrag des Reaktors gaschromatographisch analysiert. In Tabelle 5 sind die Fahrbedingungen, Eduktzusammensetzungen und die Analyse, getrennt nach Fraktionen der C₄- und der C₈-Fraktion, zusammengestellt.

Es wurden zwei Versuche (4a, 4b) mit unterschiedlich zusammengesetzten Zuläufen durchgeführt. Bei jedem Versuch wurde frischer Katalysator verwendet.

**Tabelle 5 - Reaktionsbedingungen und Analyse der Isobutenoligomerisierung**

| **Versuch Nr.** | | 4a | 4b |
|---|---|---|---|
| Temp. Wärmebad | (°C) | 100 | 100 |
| Zulauf Reaktor | (kg/h) | 0.509 | 0.480 |

| **Analyse Zulauf** | | | |
|---|---|---|---|
| Propen | (%) | 0.00 | 0.00 |
| iso-Butan | (%) | 2.75 | 0.04 |
| n-Butan | (%) | 8.52 | 13.85 |
| trans-Buten | (%) | 8.98 | 17.23 |
| 1-Buten | (%) | 29.00 | 19.17 |
| iso-Buten | (%) | 44.40 | 40.00 |
| cis-Buten | (%) | 5.76 | 9.37 |
| Rest | (%) | 0.60 | 0.35 |

| **Austrag Analyse C4-Fraktion (auf 100% C4 normiert)** | | | |
|---|---|---|---|
| iso-Butan | (%) | 3.41 | 0.06 |
| n-Butan | (%) | 10.80 | 20.17 |
| trans-Buten | (%) | 12.31 | 26.33 |
| 1-Buten | (%) | 34.07 | 24.15 |
| iso-Buten | (%) | 30.45 | 14.18 |
| cis-Buten | (%) | 8.37 | 14.77 |
| Rest | (%) | 0.60 | 0.35 |
| | | | |
| **Umsatz Isobuten** | **(%)** | **45.16** | **75.22** |
| C8-Selektivität | (%) | 87.71 | 78.01 |

| **Analyse der C8-Fraktion (auf 100% C8 normiert)** | | | |
|---|---|---|---|
| 2,4,4-Tri-Me-Penten | (%) | 94.48 | 90.53 |

### Beispiel 5 - MTBE-Synthese in Festbettreaktoren

Für die Versuche wurde ein Aufbau analog zur Isobutenoligomerisierung genutzt. Der Reaktor bestand aus einem zur Spirale gewickelten Rohr, das von Außen durch ein Ölbad temperiert wurde. Der vordere Teil (ca. das erste Viertel) des Reaktors wurde nicht mit Katalysator gefüllt, um den Zulauf erst auf die gewünschte Temperatur vorzuheizen. In den hinteren Teil wurden in Versuch 5a 125 ml, in Versuch 5b 94 ml Ionentauscher eingehüllt. Der Reaktor wurde über das Ölbad auf (isotherm) 50 °C temperiert.

Als Katalysator wurde Amberlyst 15 eingesetzt. Der Druck der Anlage betrug 8 bara. Als Zulauf wurden die C₄-Fraktionen aus Beispiel 3 eingesetzt. Analysen und Zulaufmengen sind in Tabelle 6 angegeben. Prozentangaben sind als Massen-% zu lesen.

**Tabelle 6 - Zuläufe und Analysen der MTBE Festbettversuche**

| | | **Versuch 5a** | | **Versuch 5b** | |
|---|---|---|---|---|---|
| Zulauf C4 | (g/h) | 100 | | 100 | |
| Zulauf Methanol | (g/h) | 7.7 | | 6.4 | |

| | | **Analyse Zulauf** | **Analyse Austrag** | **Analyse Zulauf** | **Analyse Austrag** |
|---|---|---|---|---|---|
| Methanol | (%) | 7.15 | 1.26 | 6.03 | 1.11 |
| iso-Buten | (%) | 10.87 | 0.57 | 9.15 | 0.57 |
| MTBE | (%) | 0.00 | 16.19 | 0.00 | 13.50 |
| n-Butan | (%) | 16.19 | 16.17 | 14.81 | 14.82 |
| iso-Butan | (%) | 3.84 | 3.86 | 3.52 | 3.51 |
| 1-Buten | (%) | 4.48 | 4.49 | 40.07 | 40.10 |
| cis-Buten | (%) | 17.84 | 17.84 | 10.04 | 10.02 |
| trans-Buten | (%) | 39.38 | 39.39 | 15.82 | 15.83 |
| Rest | (%) | 0.24 | 0.23 | 0.56 | 0.55 |

### Beispiel 6 a - Reaktivdestillation

Der schematische Aufbau der Reaktivdestillationskolonne ist in Figur 1 bzw. 2 widergegeben. Der Durchmesser der Kolonne betrug 312,7 mm. Im oberen Teil der Kolonne wurden ca. 2 m Montz-Pak A3-500 als Verstärkungsteil installiert. Darunter, ebenfalls im Verstärkungsteil der Kolonne befanden sich ca. 11,4 m Katapak-SP 12. Der untere Teil der Kolonne bestand aus ca. 3,6 m Montz-Pak A3-500. Die Zulaufposition befand sich im oberen Drittel der unteren Montz-Pak A3-500.

Als Katalysator in der Katapak-SP 12 wurde Amberlyst 15 eingesetzt. Der Druck der Anlage betrug 7,5 bara.

Als Zulauf für die Kolonne wurde aus Produktströmen einer großtechnischen Anlage ein Mischung erstellt. Dabei wurde die Isobuten-, MTBE- und Methanolkonzentration möglichst identisch zu denen der Austräge der Laborreaktoren aus Beispiel 5 eingestellt. Die Zusammensetzung der restlichen C₄-Kohlenwasserstoffe ist vergleichbar zu den Austrägen der Laborreaktoren aus Beispiel 5.

Die Zulaufmenge betrug 700 kg/h. Es wurde ein Rücklaufverhältnis von 1,09 eingestellt. Als Destillat wurden 578,8 kg/h, als Sumpfprodukt 121,2 kg/h gewonnen. Prozentangaben sind als Massen-% zu lesen.

Im Destillat wurde ein iso-Buten-Gehalt von 136 ppm gemessen.

| **Versuch 6a** | | **Analyse Zulauf** | **Analyse Destillat** | **Analyse Sumpf** |
|---|---|---|---|---|
| Methanol | (%) | 1.26 | 1.14 | 0.00 |
| iso-Buten | (%) | 0.57 | 0.01 | 0.00 |
| MTBE | (%) | 16.19 | 0.00 | 95.82 |
| n-Butan | (%) | 14.17 | 17.24 | 0.01 |
| iso-Butan | (%) | 4.995 | 6.08 | 0.00 |
| 1-Buten | (%) | 7.855 | 9.57 | 0.00 |
| cis-Buten | (%) | 16.84 | 20.49 | 0.03 |
| trans-Buten | (%) | 37.39 | 45.48 | 0.03 |
| Rest | (%) | 0.73 | 0.00 | 4.11 |

### Beispiel 6 b - Reaktivdestillation

In der gleichen Reaktivdestillationsanlage wie in Beispiel 6 a beschrieben wurde ein Versuch mit einem Edukt-Strom anderer Zusammensetzung durchgeführt.

Als Katalysator wurde ebenfalls Amberlyst 15 eingesetzt. Der Druck der Anlage betrug 7,5 bara.

Die Zulaufmenge betrug 700 kg/h. Es wurde ein Rücklaufverhältnis von 1,09 eingestellt. Als Destillat wurden 595,5 kg/h, als Sumpfprodukt 104,5 kg/h gewonnen. Prozentangaben sind als Massen-% zu lesen.

Im Destillat wurde ein iso-Buten-Gehalt von 102 ppm gemessen.

| **Versuch 6b** | | **Analyse Zulauf** | **Analyse Destillat** | **Analyse Sumpf** |
|---|---|---|---|---|
| Methanol | (%) | 1,11 | 0,93 | 0,00 |
| iso-Buten | (%) | 0,57 | 0,01 | 000 |
| MTBF | (%) | 13,50 | 0,00 | 96,28 |
| n-Butan | (%) | 12,84 | 15,09 | 0,01 |
| iso-Butan | (%) | 5,49 | 6,46 | 0,00 |
| 1-Buten | (%) | 36,77 | 43,23 | 0,01 |
| cis-Buten | (%) | 11,13 | 13,07 | 0,03 |
| trans-Buten | (%) | 18,05 | 21,21 | 0,02 |
| Rest | (%) | 0,55 | 000 | 3,65 |

### Die Bezeichnungen in den Fig. 1 und Fig. 2 haben folgende Bedeutungen:

1. Isobuten-haltiger C₄-Kohlenwasserstoffstrom
2. Butadien-Hydrierung
3. Wasserstoff
4. Rest-Butadien Selektiv-Hydrierung
5. Wasserstoff
6. Buten-Oligomerisierung
7. Destillation zur Oligomerenabtrennung
8. Butenoligomere
9. MTBE-Festbettstufe
10. Methanol
11. Reaktivdestillationskolonne
12. Reaktive Packung der Reaktivdestillationskolonne
13. Isobuten-freier n-Buten-Strom
14. MTBE
15. Butadien-Extraktion
16. Butadien
17. Abgasstrom
18. Abgasstrom

## Patentansprüche

1. Verfahren zur gekoppelten Herstellung von Butenoligomeren und tert.-Butylethem aus Isobuten-haltigen C₄-Strömen durch
a) teilweise Oligomerisierung der Isobuten-haltigen C₄-Ströme an einem sauren Katalysator zu Butenoligomeren und anschließend
b) sauer katalysierte Veretherung des restlichen Isobuten mit einem Alkohol zu tert.-Butylethern,
**dadurch gekennzeichnet,**
**dass** die in den Isobuten-haltigen C₄-Strömen enthaltenen mehrfach ungesättigten. Kohlenwasserstoffe vor der Oligomerisierung in Stufe a) katalytisch hydriert werden, dass als saurer Katalysator in Stufe a) ein Ionenaustauscher eingesetzt wird, dessen Protonen teilweise gegen Metallionen der 1. bis 12. Gruppe des Periodensystems ausgetauscht wurden, und dass die sauer katalysierte Veretherung in Stufe b) in mindestens zwei Reaktionsstufen durchgeführt wird, wobei mindestens die letzte Reaktionsstufe als Reaktivdestillation durchgeführt wird und dass die in Stufe a) gewonnenen Butenoligomeren vor der sauer katalysierten Veretherung in Stufe b) abgetrennt werden.

2. Verfahren nach Anspruch 1,
**dadurch gekennzeichnet,**
**dass** 1 bis 60 % der Protonen des in Stufe a) verwendeten Ionenaustauschers gegen Metallionen ausgetauscht sind.

3. Verfahren nach Anspruch 1 oder 2,
**dadurch gekennzeichnet,**
**dass** die Oligomerisierung in Stufe a) bis zu einem Isobutenumsatz von 50 bis 95 % durchgeführt wird.

4. Verfahren nach einem der Ansprüche 1 bis 3,
**dadurch gekennzeichnet,**
**dass** die Oligomerisierung in Stufe a) in Anwesenheit eines Moderators durchgeführt wird.

5. Verfahren nach Anspruch 4,
**dadurch gekennzeichnet**,
das als Moderator MTBE, TBA, Methanol oder Wasser in einem Molverhältnis von 0.01 bis 5 pro Mol Isobuten eingesetzt wird.

6. Verfahren nach einem der Ansprüche 1 bis 5,
**dadurch gekennzeichnet,**
**dass** in Stufe b) als Alkohol Methanol oder Ethanol eingesetzt wird.

7. Verfahren nach einem der Ansprüche 1 bis 6,
**dadurch gekennzeichnet,**
**dass** die Hydrierung der mehrfach ungesättigten Verbindungen in mindestens zwei Reaktionsstufen erfolgt, wobei mindestens die letzte Reaktionsstufe in Anwesenheit von 0,05 - 100 wppm CO durchgeführt wird.

## Claims

1. A process for coproducing butene oligomers and tert-butyl ethers from isobutenic C₄ streams by
a) partly oligomerizing the isobutenic C₄ streams over an acidic catalyst to give butene oligomers and subsequently
b) etherifying the remaining isobutene with an alcohol under acid catalysis to give tert-butyl ethers,
**characterized in that**
the polyunsaturated hydrocarbons contained in the isobutenic C₄ streams are catalytically hydrogenated before the oligomerization in stage a), **in that** the acidic catalyst used in stage a) is an ion exchanger whose protons have partly been exchanged for metal ions of groups 1 to 12 of the Periodic Table, and **in that** the etherification is carried out under acid catalysis in stage b) in at least two reaction stages, of which at least the last reaction stage is carried out as a reactive distillation, and **in that** the butene oligomers obtained in stage a) are removed before the acid-catalyzed etherification in stage b).

2. The process as claimed in claim 1,
**characterized in that**
from 1 to 60% of the protons of the ion exchanger used in stage a) have been exchanged for metal ions.

3. The process as claimed in claim 1 or 2,
**characterized in that**
the oligomerization in stage a) is carried out up to an isobutene conversion of from 50 to 95%.

4. The process as claimed in any of claims 1 to 3,
**characterized in that**
the oligomerization in stage a) is carried out in the presence of a moderator.

5. The process as claimed in claim 4,
**characterized in that**
the moderator used is MTBE, TBA, methanol or water in a molar ratio of from 0.01 to 5 per mole of isobutene.

6. The process as claimed in any of claims 1 to 5,
**characterized in that**
the alcohol used in stage b) is methanol or ethanol.

7. The process as claimed in any of claims 1 to 6,
**characterized in that**
the polyunsaturated compounds are hydrogenated in at least two reaction stages, of which at least the last reaction stage is carried out in the presence of 0.05 - 100 ppm by weight of CO.

## Revendications

1. Procédé de fabrication couplée d'oligomères de butène et d'éthers de tert.-butyle à partir de courants en C₄ contenant de l'isobutène, par
a) oligomérisation partielle des courants en C₄ contenant de l'isobutène sur un catalyseur acide en oligomères de butène, puis
b) éthérification sous catalyse acide de l'isobutène restant avec un alcool en éthers de tert.-butyle, **caractérisé en ce que**
les hydrocarbures polyinsaturés contenus dans les courants en C₄ contenant de l'isobutène sont hydrogénés catalytiquement avant l'oligomérisation de l'étape a), **en ce qu'**un échangeur d'ions, dont les protons ont été partiellement remplacés par des ions métalliques des groupes 1 à 12 du tableau périodique, est utilisé en tant que catalyseur acide à l'étape a), et **en ce que** l'éthérification sous catalyse acide à l'étape b) est réalisée en au moins deux étapes de réaction, au moins la dernière étape de réaction étant réalisée sous la forme d'une distillation réactive, et **en ce que** les oligomères de butène obtenus à l'étape a) sont séparés avant l'éthérification sous catalyse acide à l'étape b).

2. Procédé selon la revendication 1, **caractérisé en ce que** 1 à 60 % des protons de l'échangeur d'ions utilisé à l'étape a) sont remplacés par des ions métalliques.

3. Procédé selon la revendication 1 ou 2, **caractérisé en ce que** l'oligomérisation à l'étape a) est réalisée jusqu'à une conversion de l'isobutène de 50 à 95 %.

4. Procédé selon l'une quelconque des revendications 1 à 3, **caractérisé en ce que** l'oligomérisation à l'étape a) est réalisée en présence d'un modérateur.

5. Procédé selon la revendication 4, **caractérisé en ce que** du MTBE, du TBA, du méthanol ou de l'eau est utilisé en tant que modérateur en un rapport en moles de 0,01 à 5 par mole d'isobutène.

6. Procédé selon l'une quelconque des revendications 1 à 5, **caractérisé en ce que** du méthanol ou de l'éthanol est utilisé à l'étape b) en tant qu'alcool.

7. Procédé selon l'une quelconque des revendications 1 à 6, **caractérisé en ce que** l'hydrogénation des composés polyinsaturés a lieu en au moins deux étapes de réaction, au moins la dernière étape de réaction étant réalisée en présence de 0,05 à 100 ppm en poids de CO.
